**Europäisches Patentamt**

**European Patent Office**

**Office européen des brevets**

(19)

(11) Veröffentlichungsnummer: **0 137 460**
**B1**

(12) # EUROPÄISCHE PATENTSCHRIFT

(45) Veröffentlichungstag der Patentschrift:
23.07.86

(51) Int. Cl.⁴: **C 07 C 91/44,** C 07 D 263/58

(21) Anmeldenummer: **84111935.7**

(22) Anmeldetag: **05.10.84**

(54) Verfahren zur Herstellung von 5-Chlor-2-aminophenol.

(30) Priorität: 12.10.83 DE 3337043

(43) Veröffentlichungstag der Anmeldung:
17.04.85 Patentblatt 85/16

(45) Bekanntmachung des Hinweises auf die Patenterteilung:
23.07.86 Patentblatt 86/30

(84) Benannte Vertragsstaaten:
CH DE FR GB LI

(56) Entgegenhaltungen:
EP-A-0 037 352

CHEMICAL ABSTRACTS, Band 88, Nr. 5, 30. Januar 1978, Seite 503, Nr. 37784b, Columbus, Ohio, US; JOURNAL OF THE AMERICAN CHEMICAL SOCIETY, Band 71, Nr. 4, 23. April 1949, Seiten 1265-1268; W.J. CLOSE et al.: "The analgesic activity of some benzoxazolone derivatives"

(73) Patentinhaber: HOECHST AKTIENGESELLSCHAFT, Postfach 80 03 20, D-6230 Frankfurt am Main 80 (DE)

(72) Erfinder: Arndt, Otto, Dr., Frankfurter Strasse 38, D-6238 Hofheim am Taunus (DE)
Erfinder: Papenfuhs, Theodor, Dr., Heinrich-Bleicher- Strasse 40, D-6000 Frankfurt am Main 50 (DE)

EP 0 137 460 B1

## Beschreibung

Die Erfindung betrifft ein Verfahren zur Herstellung von 5-Chlor-2-aminophenol durch Chlorieren von Benzoxazolon (Benzoxazolin-2-on) in Wasser zum 6-Chlorbenzoxazolon und saure hydrolytische Spaltung der letztgenannten Verbindung zum 5-Chlor-2-aminophenol. Die Chlorierung des Benzoxazolons in organischen Lösemitteln und die alkalische hydrolytische Spaltung des 6-Chorbenzoxazolons sind als Einzelreaktionen bekannt. (J. Am. Chem. Soc. 71 (1949) 1266; EP 0 037 352; SU 0 245 111; SU 325 845.)

Die literaturbekannten Chlorierungen werden ausschließlich in organischen Lösemitteln (Chlorkohlenwasserstoffen, Dioxan, Eisessig) mit elementarem Chlor oder Sulfurylchlorid ausgeführt. Der Einsatz von Chlorrierungsmitteln, die Wasser als Verdünnungsmittel enthalten (Chlorbleichlauge, wäßrige Salzsäure/wasserstoffperoxid) ist in diesem Zusammenhang nicht bekannt, da offenbar Wasser als ungünstig angesehen wird. In einem Fall wird zwar dem organischen Lösemittel (Dioxan) wasser zugesetzt (EP 0 037 352), aber die ausschließliche Verwendung von Wasser ist nicht bekannt. Hinsichtlich eines ähnlichen Falls; (Chlorierung von O,N-Diacetyl-2-aminophenol wird von Theilacker, Ber. Dtsch. chem. Ces. 71 2066 (1938), sogar ausdrücklich darauf hingewiesen, daß wasserfreie Lösemittel zu verwenden sind, um eine Verseifung der 0-Acetylgruppe zu vermeiden. In der letztgenannten Literaturstelle wird auch die anschließende Verseifung des chlorierten 0,N-Di-acetyl-2-aminophenols (hydrolytische Abspaltung von 2-Mol Essigsäure) mit heißer konzentrierter Salzsäure beschrieben.

Für die hydrolytische Ringöffnung des Imidazolon-Rings im 6-Chlorbenzoxazolon zum 5-Chlor-2-aminophenol wird in der Literaturjedoch nur die alkalische Verseifung mit beispielsweise wäßriger Natronlauge erwähnt (J. Am. Chem. Soc. 71 1. c., SU 339 846). In den deutschen Patentschriften 2 935 629 und 2 939 056 wird eine andere Möglichkeit zur Herstellung von 5-Chlor-2-aminophenol beschrieben, wobei 2,4-Dichlornitrobenzol in dipolar aprotischem Lösemittel (z.B. Dimethylsulfoxid) bzw. In einem mit Wasser nicht mischbaren organischen Lösemittel in Gegenwart eines Phasentransferkatalysators mit 50 % iger wäßriger Kalilauge in 5-Chlor-2-nitrophenol überführt wird. Letzteres wird dann katalytisch oder mit anderen Reduktionsmitteln zum Amin reduziert (DDT 27 145, 1961).

Wie ersichtlich erfolgen die zum Stand der Technik zählenden Chlorierungen in organischen Lösemitteln. Die Regeneration der organischen Lösemittel ist technisch aufwendig. Außerdem handelt es sich in den meisten Fällen um gewerbetoxikologisch bedenkliche Lösemittel. So verursachen Chlorkohlenwasserstoffe Abwasser- und Abluftprobleme, 1,4-Dioxan gilt als cancerogen (vgl. Abschnitt III B der MAK-Werte-Liste 1982). Ein weiterer Nachteil ist die Bildung von Abgasen, die Chlorwasserstoff, Schwefeldioxid und Chlor enthalten (SU 325 845).

Offensichtlich wird nach dem Stand der Technik der Einsatz von Wasser gemieden. Es wird sogar ein Vorurteil dagegen aufgebaut (siehe Theilacker 1. c.). Weiterhin ist in der Literatur kein Verfahren bekannt gemacht worden, bei dem 5-Chlor-2-aminophenol aus Benzoxazolon hergestellt wird. Bei der Chlorierung von Benzoxazolon besteht die Gefahr einer Überchlorierung zum 4,6-Dichlorbenzoxazolon und 4,5,6-Trichlorbenzoxazolon (SU 396 338). Die Überchlorierung erfolgt bei der Chlorierung in organischen Lösemitteln sehr leicht und ist, abgesehen von der Anwendung eines Überschusses an Chlorierungsmittel, abhängig von den Verfahrensfaktoren, die einen Einfluß auf die Selektivität der Chlorierung haben. Einen besonders günstigen Einfluß auf die Selektivität kann man nach den üblichen Erfahrungen erwarten von einer ausreichend hohen Löslichkeit der zu chlorierenden Ausgangskomponente und von der Vermeidung von Festkörpersinschlüssen der Ausgangskomponente durch das Chlorierungsprodukt. Diese Voraussetzungen sind offenbar nach dem Stand der Technik für die genannten Lösemittel erfüllt, jedoch für Wasser als nicht gegeben angesehen worden.

In der Literatur findet sich kein Beispiel, bei welchem die Chlorierung des Benzoxazolons in einem Medium stattfindet, in dem Benzoxazolon und 6-Chlorbenzoxazolon unlöslich sind. So wurde auch in der Literatur noch kcine Chlorierung von Benzoxazolon in Wasser als alleinigem Verdünnungsmittel beschrieben.

Es wurde nun gefunden, daß man 5-Chlor-2-aminophenol in hoher Selektivität herstellen kann, indem man Benzoxazolon in Wasser a) mit Alkalimetall- oder Erdalkalimetallhypochloriten bei $p_H$-Werten von 0 bis 3, vorzugsweise von 1 bis 2, und bei Temperaturen von 50 bis 70°C, vorzugsweise bei 60°C, oder b) mit Chlorionen und anorganischen Peroxiden, wie beispielsweise Wasserstoffperoxid, in Gegenwart von verdünnten bis konzentrierten, vorzugsweise konzentrierten Mineralsäuren, bei Temperaturen von 50 bis 100°C, vorzugsweise 80 bis 90°C, zum 6-Chlorbenzoxazolon umsetzt und dieses ohne oder nach Zwischenisolierung mit Mineralsäuren mittlerer bis hoher Konzentration bei Temperaturen von 100 - 170°C hydrolysiert.

Geeignete Mineralsäuren bei der Chlorierung mittels Wasserstoffperoxid sind beispielsweise Salzsäure, Schwefelsäure oder Phosphorsäure. Bei der Verwendung von Salzsäure beträgt deren Konzentration in der Reaktionsmischung mindestens 20 %, vorzugsweise 30 %. Bei der Chlorierung mittels Wasserstoffperoxid ist die Verwendung von Salzsäure als Mineralsäure dann zwingend, wenn keine anderen Chlorionenspender, welche die Chlorionen in für die Chlorierungsreaktion ausreichender Menge zur Verfügung stellen, vorhanden sind. Solche anderen Chlorionenspender sind beispielsweise Alkalimetallchloride, vorzugsweise Natrium- oder Kaliumchlorid, die in einer Menge von mindestens 2 Mol pro Mol zu chlorierendem Benzoxazolon, zweckmäßiger Weise einem mäßigen Überschuß zugegen sind. Bei der Anwendung des Systems Wasserstoffperoxid/Salzsäure beträgt die Konzentration der Salzsäure in der Reaktionsmischung zweckmäßigerweise 10 - 37 %, vorzugsweise 20 - 30 %.

Die saure hydrolytische Spaltung des in erster Stufe erhaltenen 6-Chlorbenzoxazolons zum 5-Chlor-2-amino-phenol kann beispielsweise in Salzsäure einer Konzentration von z.B. 15 - 37 %, Schwefelsäure einer Konzentration von 30 - 70 % oder Phosphorsäure einer Konzentration von 40 bis 80 % vorgenommen werden. Die Hydrolyse kann unter Druck oder bei ausreichend hoher Säurekonzentration auch bei Normaldruck erfolgen. Die angegebenen Säurekonzentrationen beziehen sich auf die Säurekonzentration in der Reaktionsmischung.

Bei der Chlorierung mittels Hypochlorit kann handelsübliche Chlorbleichlauge verwendet werden. Unter Chlorbleichlauge ist hierbei eine wäßrige Lösung von Natriumhypochlorit und Natriumchlorid zu verstehen, die durch Einleiten von Chlor in kalte wäßrige Natronlauge hergestellt werden kann. Die technische Chlorbleichlauge ist 13 - 13 5 gewichtsprozentig was bedeutet, daß diese Chlorbleichlauge 160 - 165 g wirksames Chlor im Liter enthält. Neben dieser technischen Chlorbleichlauge, die beim Verfahren der vorliegenden Erfindung bevorzugt eingesetzt wird, können verfahrensgemäß auch Chlorbleichlaugen niedrigerer Konzentrationen verwendet werden. (Gmelins Handbuch der anorganischen Chemie 6 (1927) Seite 293; Ullmanns Encyklopädie der technischen Chemie 9, 4. Auflage (1975), Seite 544). Bei der Chlorierung mittels Chlorbleichlauge ist es beim erfindungsgemäßen Verfahren zweckmäßig, einen Überschuß bis maximal 20 Mol-% über die äquivalente Menge hinaus anzuwenden.

Bei der Chlorierung mittels Wasserstoffperoxid/Chlorionen wendet man das Wasserstoffperoxid in äquivalenter Menge an, wobei ein kleiner Überschuß, beispielsweise von 3 bis 10 Mol-%, vorzugsweise von 3 Mol-%, zweckmäßig sein kann.

Die Anwendung von Katalysatoren bei der Chlorierungsreaktion, wie beispielsweise $JCl_3$ oder $FeCl_3$, ist möglich, aber nicht zwingend. Bei der Anwendung von $FeCl_3$ als Katalysator müßte ohnehin die auf NaClO und $H_2O_2$ zersetzende Wirkung von $Fe^{3+}$-Ionen beachtet werden.

Das in erster Stufe erhaltene 6-Chlorbenzoxazolon kann in jedem Fall vor der hydrolytischen Spaltung isoliert werden. Eine Zwischenisolierung bei der Chlorierung mittels Alkalimetallhypochlorit (Chlorbleichlauge) ist allerdings zwingend, während im Falle der Chlorierung mittels Wasserstoffperoxid/Chlorionen eine Zwischenisolierung nicht erforderlich ist. In manchen Fällen kann eine Zwischenisolierung von Vorteil sein, beispielsweise um eine größere Reinheit des Endprodukts (5-Chlor-2-aminophenol) zu erzielen.

Nachdem die Chlorierung von Benzoxazolon gemäß der vorstehend zitierten Literatur bisher nur in organischen Lösemitteln durchgeführt wurde, muß es als überraschend erachtet werden, daß die Chlorierung in Wasser ohne Schwierigkeiten mit ausreichender Selektivität durchführbar ist, besonders aus der Sicht der weiter oben genannten Nachteile, die ein hinsichtlich des zu chlorierenden Produkts nur wenig lösendes Verdünnungsmittel mit sich bringt.

Damit wird das in der Literatur speziell für die Chlorierung von Benzoxazolon immanente Vorurteil gegenüber dem einfachsten Medium, nämlich Wasser, durch die vorliegende Erfindung aufgehoben. Die Anwendung von Wasser hat gegenüber dem Einsatz organischer Lösemittel zusätzlich den Vorteil, daß sie im Rahmen einer bevorzugten Ausführungsform ein "Eintopfverfahren" (ohne Zwischenisolierung von 6-Chlorbenzoxazolon) ermöglicht. Die in der Literatur für die Chlorierung genannten Lösemittel (Tetrachlorethan, Dioxan, Essigsäure) sind für die anschließende hydrolytische Öffnung des Imidazolonringes zum o-Aminophenol aus technischen, chemischen und toxikologischen Gründen ungeeignet und müssen vorher abgetrennt werden. So erfordern sie beispielsweise einen Spezial-Emailkessel mit einer Druckauslegung von über 10 bar. Nebenreaktionen in der Art von Acylierung und Alkylierung des bifunktionellen o-Aminophenols sind nicht auszuschließen.

Ein weiterer Vorteil des erfindungsgemäßen Verfahrens ist, daß kein Chlor- und HCl-haltiges Abgas entsteht. Somit entfällt eine technisch aufwendige Abgasreinigung. Weiterhin wurde gefunden, daß das Verfahren besonders geeignet ist zur Abtrennung der nie völlig ausbleibenden Nebenkomponenten, weil es die abgestuften pK-Werte der einzelnen Komponenten zu nutzen vermag. Vor der Hauptfällung des 5-Chlor-2-aminophenols bei pH 3,5 - 7 erfolgt eine Klärung bei pH 2,2 - 3,0, wobei der größte Teil der schwächer basischen Nebenprodukte (höher chlorierte Komponenten) abgetrennt wird.

Das O-Aminophenol aus dem nicht chlorierten Anteil von Benzoxazolon bleibt wegen der geringen Menge, in der es anfällt, in der Mutterlauge gelöst und wird somit bei der Hauptfällung abgetrennt. Nach dem erfindungsgemäßen Verfahren wird nahezu weißes 5-Chlor-2-amino-phenol in einer Reinheit von mindestens 90 bis 97 % und einer Ausbeute von ca. 70-80 %, bezogen auf Benzoxazolon, erhalten. Es ist je nach Anforderung in dieser Form oder nach einer Druckumlösung in Wasser in noch reinerer Form für die Verwendung als Zwischenprodukt beispielsweise zur Herstellung von Pflanzenschutzmitteln geeignet.

Nachstehend seien noch Einzelheiten bzw. bevorzugte Ausführungen des erfindungsgemäßen Verfahrens beschrieben:

Bei der Chlorierung mit Chlorbleichlauge bei niederer Säurekonzentration (z.B. bei pH 0 - 3), empfiehlt es sich, das als Festkörper suspendierte Benzoxazolon vorher durch geeignete Maßnahmen in eine feinverteilte Form zu überführen und dann mit dem Chlorierungsmittel (Chlorbleichlauge) umzusetzen. Dazu eignen sich die üblichen Methoden, mit denen eine Feinverteilung zu erzielen ist, wie beispielsweise Lösen des Benzoxazolons mit einer äquivalenten Menge Natronlauge zum Natriumsalz und rasche Wiederausfällung des Benzoxazolons durch Einlaufenlassen der alkalischen Lösung in intensiv gerührte, kalte Mineralsäure (z.B. verdünnte wäßrige Salzsäure).

Vor bzw. während der Chlorierung können eine oder mehrere Naßvermahlungen (z.B. durch Umpumpen) vorgenommen werden. Auch intensiv wirkende Rührorgane, wie beispielsweise Hochleistungs-Dispergier- und Emulgiergeräte, die für eine Kornverkleinerung und gute Durchmischung sorgen, sind geeignet.

Weiterhin ist der Zusatz von Detergentien (beispielsweise auf Basis Naphthalinsulfosäure-Formaldehyd-Kondensat) hilfreich.

Die vorstehend genannten Maßnahmen zur Feinverteilung sind auch entbehrlich. Sie verbessern nur die Selektivität der Chlorierung. Sie sind speziell dann entbehrlich, wenn auf eine andere Art und Weise für eine bessere Feinverteilung gesorgt ist. So geht beispielsweise das Benzoxazolon bei Anwendung einer z.B. 30 %igen Salzsäure weitgehend in Lösung. Das ist von Bedeutung bei der Anwendung des Systems Wasserstoffperoxid/Salzsäure als Chlorierungsmittel. Hierbei reagiert das Chlor in statu nascendi (Zinke, Ber. Dtsch. chem. Ges. 58, 330 (1925), Seikel, Org. Synth. Coll. Vol. III, 262 (1956), $H_2O$ und seine Derivate, Weigert, Verlag Hüthig 1978, 103). Die Chlorierung mit Wasserstoffperoxid/Salzsäure ist für das Konzept eines Eintopfverfahrens besonders geeignet, da anders als bei Einsatz von Chlorbleichlauge, keine anorganischen Salze anfallen. Abgesehen von einer Entlastung des Abwassers von anorganischen Salzen wird insbesondere die Möglichkeit geboten, wesentlich konzentriertere Suspensionen des 6-Chlorbenzoxazolons zur Hydrolyse einzusetzen.

Dadurch wird die Raumzeitausbeute erheblich gesteigert und die Kesselkapazitäten werden besser genutzt. Der Anteil an Säure, der zur Aufstärkung der Chlorbenzoxazolon-Suspension auf die für die Hydrolyse notwendige Säurekonzentration benötigt wird, entfällt, was eine weitere Salzentlastung bedeutet.

Im Falle der Anwendung von Chlorbleichlauge als Chlorierungsmittel empfiehlt es sich, das entstehende Natriumhydroxid durch Zulauf von Mineralsäure in bekannter Weise zu neutralisieren, um den optimalen pH-Bereich (0-3) aufrechtzuhalten.

Die Gegenwart von $Fe^{3+}$-Ionen bei der Hydrolyse des 6-Chlorbenzoxazolons zum o-Aminophenol, welches mit $Fe^{3+}$-Ionen stark gefärbte Komplexe bildet und in ihrer Gegenwart thermisch labiler ist, ist zu vermeiden.

Es ist ein weiterer Vorteil des erfindungsgemäßen Verfahrens, daß bei der Chlorierungsreaktion keine Katalysatoren eingesetzt werden müssen, die überdies noch bei der nachfolgenden Hydrolyse des 6-Chlorbenzoxazolons zum o-Aminophenol stören könnten.

Für das "Eintopfverfahren" ist die Hydrolyse in konzentrierten wäßrigen Mineralsäuren die Methode der Wahl. Bei Verwendung von Salzsäure dient diese dann nicht nur als Chlorierungsmittel, sondern auch als Mittel für die Ringspaltung. Grundsätzlich ist auch eine alkalische Ringspaltung denkbar; diese setzt aber eine Zwischenisolierung des 6-Chlorbenzoxazolons voraus und bietet somit keine ersichtlichen Verfahrensvorteile.

Bei der sauren Ringspaltung entsteht Kohlendioxid, das jedoch beim Arbeiten unter Druck zu ca. 70 % in der Reaktionslösung gelöst bleibt. Es kann auch über ein Druckhalteventil abgelassen werden, wobei zu berücksichtigen ist, daß mit abnehmendem Druck (z.B. C 5 bar) zwar die Azeotrop-Konzentration der Salzsäure steigt, aber mehr Chlorwasserstoff durch das Entweichen von gelöstem $CO_2$ ausgetrieben wird. Der Druckanstieg im geschlossenen Gefäß ist im allgemeinen auf ca. 20 bar begrenzt. Um den Chlorwasserstoff-Verlust bei der $CO_2$-Entspannung zu verringern, empfiehlt es sich, die Konzentration der Salzsäure nicht wesentlich über 20 % steigern. Für eine 20 - 30 %ige Salzsäure bzw. 30 - 50 %ige Schwefelsäure haben sich Temperaturen von 125 - 150° C als optimal erwiesen.

Prinzipiell gilt, daß niedere Temperaturen höhere Säurekonzentrationen erfordern. Höhere Temperaturen als 170° C sind wegen möglicher Thermolabilität des 5-Chlor-2-aminophenols zu vermeiden.

Nach Beendigung der Druckzunahme (durch $CO_2$) bzw. des Abblasens von $CO_2$ über das Druckhalteventil läßt man das Rührgefäß langsam abkühlen. Man entspannt bei 25° C (Druck ca. 5 bar). Die Suspension des 5-Chlor-2-amino-phenol-Hydrochlorids (bzw. Hydrogensulfats) wird mit so viel Wasser, ggfs. in der Wärme, verdünnt, daß die nach der Neutralisation gebildeten Salze (NaCl, $Na_2SO_4$) in Lösung bleiben. (Es besteht auch die Möglichkeit, die Aniliniumsalze durch Filtration zu isolieren.) Die schwächer basischen Nebenkomponenten fallen während der Neutralisation mit Natronlauge zuerst aus und können gegebenenfalls in Gegenwart eines Filterhilfsmittels (z. B. Aktivkohle) durch Filtration bei pH 2,2 bis 3,0 abgetrennt werden.

Vor der Hauptfällung empfiehlt sich eine Überlagerung mit Stickstoff und eventuell eine prophylaktische Zugabe von beispielsweise Natriumdithionit.

Das 5-Chlor-2-aminophenol kristallisiert ab pH 3,5 aus. Man vervollständigt die Kristallisation durch langsame Zugabe von Natronlauge bis pH 7,0. Das 5-Chlor-2-amino-phenol wird durch Filtration isoliert.

Das 5-Chlor-2-aminophenol ist ein wertvolles Zwischenprodukt zur Herstellung von Pflanzenschutzmitteln. So läßt sich beispielsweise gemäß EP 0043573 daraus 2,6-Dichlorbenzoxazol herstellen, dessen Chlorsubstituent in 2-Stellung zahlreichen nucleophilen Substitutionen zugänglich ist und in die Reihe der Pflanzenschutzwirkstoffe (Herbizide Mittel) führt (EP 0062905).

Die nachstehenden Beispiele dienen zur Erläuterung des erfindungsgemäßen Verfahrens, ohne es darauf zu beschränken. Teile bedeuten Gewichtsteile.

**Beispiel 1**

a) Zu 3000 Teilen Wasser gibt man unter Rühren 548 Teile Benzoxazolon (als technisch feuchte Ware) ($F_p$-Wert des Trockenprodukts 139 - 141°C). Nach Stickstoffüberlagerung läßt man unter Rühren 486 Teile 33 %ige Natronlauge zulaufen. Die schwach alkalische Lösung wird über 20 Teilen Aktivkohlepulver durch eine Drucknutsche in ein intensiv gerührtes Gemisch aus 4000 Teilen Eis/Eiswasser, 580 Teilen 30 %iger Salzsäure und 6 Teilen Naphthalinsulfonsäure-Formaldehyd-Kondensat (Dispergiermittel) unter Stickstoffdruck hinein filtriert. Der Kohlerückstand wird mit 1000 Teilen Wasser gewaschen. Man heizt auf 60°C. Dann läßt man 2400 Teile einer 13,5 %igen Chlorbleichlauge unter Rühren bei 60°C in 5 Stunden zutropfen, wobei durch gleichzeitiges Zutropfen von 521 Teilen einer 30 %igen Salzsäure der pH-Wert der Suspension auf 1,0 gehalten wird. (Unter Chlorbleichlauge ist hierbei eine wäßrige Lösung von Natriumhypochlorit und Natriumchlorid zu verstehen, die durch Einleiten von Chlor in kalte, wäßrige Natronlauge hergestellt werden kann und die 160 - 165 g wirksames Chlor im Liter enthält.) Die Suspension wird während der Chlorierung (nach Zugabe von ca. 2100 Teilen Chlorbleichlauge) 5 Minuten mit einem Hochleistungs-Dispergier- und Emulgiergerät gerührt. Die Suspension wird noch 2 Stunden bei 60°C nachgerührt, ggfs. mit 10 Teilen einer 40 %igen Natriumhydrogensulfit-Lösung versetzt und das gebildete 6-Chlorbenzoxazolon durch Filtration isoliert.

Nach dem Waschen mit 2000 Teilen Wasser und Trocknen erhält man 555 Teile 6-Chlorbenzoxazolon (82 % d. Th., bezogen auf Benzoxazolon) in Form von 617 Teilen eines weißen Pulvers (Reingehalt 90 %) mit einem Schmelzpunkt von 185 - 188°C. Die gaschromatographisch erfaßbaren Verunreinigungen setzen sich wie folgt zusammen: 7 % Benzoxazolon, 2 % höher chlorierte Komponenten.

Die Oberflächenbestimmung nach Ströhlein ergab eine Oberfläche von 1,9 m²/g.

b) In eine m 4 1-Emailautoklav werden 2800 Teile 30 %ige eisenfreie Salzsäure vorgelegt. Dann werden 555 Teile 6-Chlorbenzoxazolon (= 617 Teile Trockenprodukt) als 40 %ige technisch feuchte Ware eingetragen (Gehalt an Salzsäure ca. 23 %). Man heizt unter Rühren auf 130°C. Das entstehende Kohlendioxid wird bei 4,5 bar über ein Druckhalteventil abgegast. Dann rührt man bei 135°C bis zur Beendigung der Kohlendioxid-Entwicklung (ca. 9 Stunden). Darauf läßt man langsam auf 25°C abkühlen und entspannt anschließend den Autoklav. Die Suspension des gewonnenen 5-Chlor-2-aminophenol-Hydro-chlorids wird mit 2200 Teilen Wasser verdünnt und dabei in einen Rührkolben überführt. Dann wird mit 2312 Teilen 33 %iger Natronlauge unter Außenkühlung auf pH 2,6 gestellt. Darauf klärt man über 90 Teilen Aktivkohlepulver und wäscht mit ca. 300 Teilen Wasser. Der Filterrückstand enthält ca. 50 Teile Nebenkomponenten.

Das Produktfiltrat wird bei 25°C mit 436 Teilen 33 % iger Natronlauge auf pH 7,0 gestellt. Die Kristallisation setzt ab pH 3,2 ein. Es empfiehlt sich, nach der Klärfiltration (siehe vorstehend) das Produktfiltrat mit Stickstoff zu überlagern und ggfs. 20 Teile Natriumdithionit spätestens dann zuzusetzen, wonn der pH den Wert 4 - 4,5 erreicht hat. Man isoliert das 5-Chlor-2-aminophenol aus der grauen Suspension durch Filtration. Nach Waschen mit 1500 Teilen Wasser und Trocknen erhält man 425 Teile 5-Chlor-2-aminophenol (74 % d. Th., bezogen auf Benzoxazolon) in Form von 452 Teilen eines grauen Kristallisats (Reingehalt 94 %) mit einem Schmelzpunkt von 147 - 149°C. Die gaschromatographisch erfaßbaren Verunreinigungen setzen sich wie folgt zusammen: 1,0 % 2-Aminophenol, < 0,1 % 4-Chlor-2-aminophenol, ca. 3 % Dichloraminophenole, Rest: Wasser und anorganische Bestandteile.

**Beispiel 2**

Zu 2000 Teilen Wasser gibt man unter Rühren 46 Teile 85 % ige Phosphorsäure, 2 Teile Naphthalinsulfonsäure-Formaldehyd-Kondensat und 548 Teile Benzoxazolon (als technisch feuchte Ware, Feuchtgehalt im vorgelegten Wasser eingerechnet).

Dann läßt man 2400 Teile einer 13,5 %igen Chlorbleichlauge unter Rühren bei 50°C in 6 Stunden zutropfen, wobei durch gleichzeitiges Zutropfen von 554 Teilen einer 30 % igen Salzsäure der pH der Suspension auf 2 - 2,5 gehalten wird.

Die Suspension wird noch 1 Stunde bei 50°C nachgerührt und das gebildete 6-Chlorbenzoxazolon durch Filtration isoliert. Nach dem Waschen mit 4000 Teilen Wasser und Trocknen erhält man 550 Teile 6-Chlorbenzoxazolon (81 % d.Th., bezogon auf Benzoxazolon) in Form von 654 Teilen eines weißen Pulvers (Reingehalt 84 %) mit einem Schmelzpunkt von 178 - 180°C.

Die gaschromatographisch erfaßbaren Verunreinigungen setzen sich wie folgt zusammen: 12 % Benzoxazolon, 3 % höher chlorierte Komponenten.

**Beispiel 3**

Man verfährt, wie in Beispiel 2 beschrieben, jedoch unter Zusatz von 2 Teilen Eisen(III)-chlorid und 2 Teilen Jodtrichlorid.

Man erhält 570 Teile 6-Chlorbenzoxazolon (84 % d. Th., bezogen auf Benzoxazolon) in Form von 670 Teilen eines weißen Pulvers (Reingehalt 85 %) mit einem Schmelzpunkt von 181 - 183°C.

Die gaschromatographisch erfaßbaren Verunreinigungen setzen sich wie folgt zusammen: 6 % Benzoxazolon, 8 % höher chlorierte Komponenten.

**Beispiel 4**

Zu 2175 Teilen 30 %iger eisenfreier Salzsäure gibt man unter Rühren 405 Teile Benzoxazolon und 10 Teile eines sekundären Alkansulfonates (als Emulgator). Man heizt auf 80°C und tropft unter Rühren bei 80 - 85°C in 4 Stunden 302 Teile 35 %iges Wasserstoffperoxid hinzu. Man rührt noch bei 80°C nach, prüft auf ggfs. noch vorhandenes Oxidationsvermögen (falls noch vorhanden, Beseitigung durch Zugabe von Sulfit), überführt die dicke Suspension des gebildeten 6-Chlorbenzoxazolons mit 500 Teilen 30 %iger eisenfreier Salzsäure in einen 4 l-Emailkessel und heizt auf 130°C. Das entstehende Kohlendioxid wird bei 4 bis 5 bar über ein Druckhalteventil abgegast. Man rührt bei 130 - 150°C bis zur Beendigung der Kohlendioxid-Entwicklung (ca. 5 Stunden). Anschließend rührt man noch 4 Stunden bei 150°C nach. Darauf läßt man langsam auf 25°C abkühlen und entspannt schließlich den Emailkessel. Die Suspension des gewonnen 5-Chlor-2-aminophenol-Hydrochlorids wird mit 2500 Teilen Wasser verdünnt und dabei in einen Rührkolben überführt.

Dann wird mit 1890 Teilen 33 %iger Natronlauge unter Außenkühlung auf pH 3,0 gestellt. Darauf klärt man über 90 Teilen Aktivkohlepulver und wäscht mit ca. 300 Teilen Wasser. Der Filterrückstand enthält 67 Teile Nebenkomponenten. Das Produktfiltrat wird auf 80°C geheizt und mit 343 Teilen 33 %iger Natronlauge auf $p_H$ 6,5 gestellt. Man läßt auf 25°C abkühlen. Es empfiehlt sich, nach der Klärfiltration (siehe vorstehend) das Produktfiltrat mit Stickstoff zu überlagern und ggfs. 20 Teile Natriumdithionit spätestens dann zuzusetzen, wenn der $p_H$ den Wert 4 - 4,5 erreicht hat. Man isoliert das 5-Chlor-2-amino-phenol aus der grauen Suspension durch Filtration.

Nach Waschen mit 1500 Teilen Wasser und Trocknen erhält man 289 Teile 5-Chlor-2-aminophenol (67 % d. Th., bezogen auf Benzoxazolon) in Form von 304 Teilen eines grauen Kristallisats (Reingehalt 95 %) mit einem Schmelzpunkt von 149 - 153°C.

Die gaschromatographisch erfaßbaran Verunreinigungen setzen sich wie folgt zusammen: 1,2 % 2-Aminophenol, < 0,1 % 4-Chlor-2-aminophenol, 0,5 % Dichloraminophenole, Rest: Wasser und anorganische Bestandteile.

**Beispiel 5**

Man verfährt, wie in Beispiel 4 beschrieben, jedoch bei erhöhter Produktkonzentration, d.h. der Einsatz von Benzoxazolon wird auf 548 Teile und der Einsatz von 35 % igem Wasserstoffperoxid entsprechend auf 390 Teile erhöht. Man versetzt mit 1714 Teilen 33 %iger Natronlauge bis $p_H$ 2,6 und klärt über Kohle. Der Filterrückstand enthält 71 Teile Nebenkomponenten. Das Produktfiltrat wird mit 394 Teilen 33 %iger Natronlauge auf $p_H$ 7,0 gestellt. Auch hier erfolgte eine Heißfällung (vgl. Beispiel 8).

Man erhält 377 Teile 5-Chlor-2-aminophenol (66 % d. Th., bezogen auf Benzoxazolon) in Form von 410 Teilen eines grauen Kristallisats (Reingehalt 92 %) mit einem Schmelzpunkt von 146 - 150°C.

Die gaschromatographisch erfaßbaren Verunreinigungen setzen sich wie folgt zusammen: 3,8 % 2-Aminophenol, <0,1 % 4-Chlor-1-aminophenol, 0,5 % Dichloraminophenole, 1,2 % unbekannte früh eluierbare Komponenten, Rest: Wasser und anorganische Bestandteile.

Wie ersichtlich, erhält man bei Anwendung einer höheren Produktkonzentration ein 5-Chlor-2-aminophenol deutlich schlechterer Qualität (vgl. Beispiel 4).

**Beispiel 6**

Man verfährt, wie in Beispiel 5 beschrieben, jedoch mit dem erhöhten Einsatz von 428 Teilen 35 %igem Wasserstoffperoxid (= 10 Mol % Überschuß im Vergleich zum Benzoxazolon). Der Filterrückstand enthält jetzt deutlich mehr, nämlich 125 Teile Nebenkomponenten.

Man erhält nur 330 Teile 5-Chlor-2-aminophenol (58 % d.Th.) in Form von 364 Teilen eines grauen Kristallisats (Reingehalt 90 %) mit einem Schmelzpunkt von 145-147°C. Wie ersichtlich, erhält man bei Anwendung eines 10 %igen Überschusses von Wasserstoffperoxid eine deutlich niedere Ausbeute von 5-Chlor-2-aminopehnol.

## Beispiel 7

a) Man verfährt, wie in Beispiel 1 beschrieben, jedoch unter Anwendung von 548 Teilen eines Benzoxazolons deutlich schlechterer Qualität (Fp: 134 - 138°C, 570 Teile Trockenprodukt, entsprechend einem Reingehalt von ca. 95%).

Der Kohlerückstand der Umfällung (beschrieben in Beispiel 1 a) enthält jetzt 27 statt 4 Teile Nebenkomponenten.

Man erhält 545 Teile 6-Chlorbenzoxazolon (80 % d. Th. bezogen auf Benzoxazolon) in Form von 605 Teilen eines hellbraunen Pulvers (Reingehalt 90%) mit einem Schmelzpunkt von 183 - 186°C.

Die gaschromatographisch erfaßbaren Verunreinigungen setzen sich wie folgt zusammen: 8 % Benzoxazolon, 1 - 2 % höher chlorierte Komponenten.

b) Man verfährt, wie in Beispiel 1 b beschrieben, jedoch unter Verwendung von nur 408 Teilen 6-Chlorbenzoxazolon ( = 453 Teile Trockenprodukt) als ca. 60 %ige technisch feuchte Ware.

Bei der Aufarbeitung werden 2394 Teile 33 %ige Natronlauge bis $p_H$ 3,0 zugesetzt. Der Kohlerückstand aus der Klärung enthält 40 Teile Nebenkomponenten. Das Produktfiltrat wird bei 80°C mit 286 Teilen 33 %iger Natronlauge auf $p_H$ 6,5 gestellt. Man isoliert das 5-Chlor-2-aminophenol durch Filtration bei 25°C. Man erhält 268 Teile 5-Chlor-2-aminophenol (62 % d.Th., bezogen auf Benzoxazolon) in Form von 276 Teilen eines hellgrauen Kristallistats (Reingchalt 97 %) mit einem Schmelzpunkt von 150 - 154°C. Die gaschromatographisch erfaßbaren Verunreinigungen setzen sich wie folgt zusammen: 0,7 % 2-Aminophenol, < 0,1 % 4-Chlor-2-aminophenol, 0,1 % Dichloraminophenole; Rest: Wasser und anorganische Bestandteile.

Der Vergleich mit Beispiel 1 (vgl. auch Beispiele 4 und 5) zeigt auch hicr den günstigen Einfluß der verdünnnten Fahrweise auf dic Qualität des Endprodukts. Dabei ist zu berücksichtigen, daß die Heißfällung gegenüber der Kaltfällung keine Verbesserung in der Qualität des Endprodukts bewirkt (vgl. hierzu Beispiel 8).

## Beispiel 8

Man verfährt, wie in Beispiel 5 beschrieben, jedoch mit dem Unterschied, daß die Fällung des 5-Chlor-2-aminophenols bei 25°C erfolgt. Man erhält 405 Teile 5-Chlor-2-aminophenol (70 % d. Th., bezogen auf Benzoxazolon) in Form von 440 Teilen eines grauen Kristallisats (Reingehalt 92 %) mit einem Schmelzpunkt von 148 - 150°C. Die gaschromatographisch erfaßbaren Verunreinigungen setzen sich wie folgt zusammen: 3,4 % 2-Aminophenol, <0,1 % 4-Chlor-2-aminophenol, 1,3 % Dichloraminophenole, ca. 1 % unbekannte höher eluierbare Komponenten, Rest: Wasser und anorganische Bestandteile. Wie ersichtlich, erbringt die in Beispiel 5 ausgeführte Heißfällung keine Verbesserung der Qualität, besonders bezüglich des Gehalts an 2-Aminophenol.

## Beispiel 9

Man verfährt, wie in Beispiel 4 beschrieben, jedoch unter Verwendung von 405 Teilen eines Benzoxazolons deutlich schlechterer Qualität (Pp: 134 - 138°C, 427 Teile Trockenprodukt, entsprechend einem Reingehalt von ca. 95 %). Die schlechtere Qualität des Benzoxazolons muß durch den höheren Einsatz von 350 Teilen 35 % igem Wasserstoffperoxid berücksichtigt werden, da die Verunreinigungen im Benzoxazolon verstärkt Wasserstoffperoxid verbrauchen.

Bei der Aufarbeitung werden 1904 Teile 33 %ige Natronlauge bis $p_H$ 3,0 zugegeben. Man klärt wie üblich mittels Kohle. Der Filterrückstand enthält 97 Teile Nebenkomponenten. Das Produktfiltrat wird bei 80°C mit 313 Teilen 33 %iger Natronlauge auf $p_H$ 6,5 gestellt. Das 5-Chlor-2-aminophenol wird bei 25°C durch Filtration isoliert. Man erhält 254 Teile 5-Chlor-2-aminophenol (59 % d. Th., bezogen auf Benzoxazolon) in Form von 262 Teilen eines dunklen Kristallisats (Reingehalt 97 %) mit einem Schmelzpunkt von 148 - 152°C.

Die gaschromalographisch erfaßbaren Verunreinigungen setzen sich wie folgt zusammen: 0,6 % 2-Aminophenol, < 0,1 % 4-Chlor-2-aminophenol, 0,2 % Dichloraminophenole, Rest: Wasser und anorganische Bestandteile.

## Beispiel 10

Man verfährt, wie in Beispiel 6 beschrieben (548 Teile Benzoxazolon, 2175 Teile 30 % ige Salzsäure, 433 Teile 35 % iges Wasserstoffperoxid), isoliert jedoch entgegen Beispiel 6 das gebildete 6-Chlorbenzoxazolon durch Filtration. Man erhält 550 Teile 6-Chlorbenzoxazolon (80 % d.Th., bezogen auf Benzoxazolon) in Form von 670 Teilen eines weißen Pulvers (Reingehalt 80 - 85 %) mit einem Schmelzpunkt von 180 - 184°C.

## Beispiel 11

**(Vergleichsbeispiel zu Beispielen 1 und 10)**
Zu 6000 Teilen Wasser und 10 Teilen Naphthalinsulfonsäure-Formaldehyd-Kondensat (Dispergiermittel) gibt man unter Rühren 548 Teile Benzoxazolon (als technisch feuchte Ware). Man rührt noch 10 Minuten mit einem Hochleistungs-Dispergier- und Emulgiergerät und versetzt mit 2 Teilen Jodtrichlorid. Man heizt auf 80°C. Dann läßt man gleichzeitig 1036 Teile 30 %ige Salzsäure und 428 Teile 35 %iges Wasserstoffperoxid bei 80°C in 5 Stunden zutropfen. Man isoliert schließlich 560 Teile eines Gemisches aus Benzoxazolon und 6-Chlorbenzoxazolon vom Schmelzpunkt 115 - 140°C.

Wie ersichtich erhält man bei der Chlorierung von Benzoxazolon mit Wasserstoffperoxid und einer nur äquivalenten Menge Salzsäure einer effektiven Konzentration von nur ca. 4 % (gegenüber einem großen Überschuß Sälzsäure einer effektiven Konzentration von 28 % gemäß Beispiel 10) ein chloriertes Benzoxazolon-Produkt, das einen stark erniedrigten Schmelzpunktbereich aufweist und in einer wesentlich geringeren Menge anfällt (560 Teile gegenüber 670 Teilen).

### Patentansprüche

1. Verfahren zur Herstellung von 5-Chlor-2-aminophenol in hoher Selektivität, dadurch gekennzeichnet, daß man Benzoxazolon in Wasscr a) mit Alkalimetall- oder Erdalkalimetallhypochloriten bei pH-Werten von 0 bis 3 und bei Temperaturen von 50 bis 70°C, oder b) mit Chlorionen und anorganischen Peroxiden in Gegenwart von verdünnten bis konzentrierten Mineralsäuren bei Temperaturen von 50 bis 100°C, zum 6-Chlorbenzoxazolon umsetzt und dieses ohne oder nach Zwischenisolierung mit Mineralsäuren mittlerer bis hoher Konzentration bei Temperaturcn von 100 - 170°C hydrolysiert.

2. Verfahren nach Anspruch 1a, dadurch gekennzeichnet, daß man das Benzoxazolon mit einer 13 - 13,5 gewichtsprozentigen Chlorbleichlauge (enthaltend etwa 160 - 165 g wirksames Chlor im Liter) umsetzt.

3. Verfahren nach Anspruch 1b, dadurch gekennzeichnet, daß man die Chlorierung des Benzoxazolons mit Wasserstoffperoxid in wäßrigmineralsaurem Medium durchführt, das einen Salzsäuregehalt von 20 - 30 % aufweist.

4. Verfahren nach Anspruch 1, dadurch gekennzeichnet, daß man die hydrolytische Spaltung des 6-Chlorbenz-oxazolons in 15 - 37 %iger Salzsäure vornimmt.

### Claims

1. A process for the preparation of 5-chloro-2-aminophenol in a high selectivity which comprises reacting benzoxazolone in water a) with an alkali metal hypochlorite or alkaline earth metal hypochlorite at pH values of 0 to 3 and at temperatures of 50 to 70°C, or b) with chlorine ions and an inorganic peroxides in the presence of dilute to concentrated mineral acids at temperatures of 50 to 100°C, to give 6-chlorobenzoxazolone and hydrolyzing this, with or without intermediate isolation, with a mineral acid of medium to high concentration at temperatures of 100-170°C.

2. The process as claimed in claim 1a, wherein the benzoxazolone is reacted with 13-13.5 per cent strength by weight chlorine bleaching liquor (containing about 160-165 g of active chlorine per liter).

3. The process as claimed in claim 1b, wherein the chlorination of the benzoxazolone is carried out with hydrogen peroxide in an aqueous mineral acid medium with a hydrochloric acid content of 20-30%.

4. The process as claimed in claim 1, wherein the hydrolytic cleavage of the 6-chlorobenzoxazolone is carried out in 15-37% strength hydrochloric acid.

### Revendications

1. Procédé pour préparer l'amino-2 chloro-5 phénol avec une grande sélectivité, procédé caractérisé en ce qu'on fait réagir la benzoxazolone dans de l'eau a) avec des hypochlorites de métaux alcalins ou de métaux alcalinoterreux, à des pH de 0 à 3 et à des températures de 50 à 70°C, ou b) avec des ions de chlore et des peroxydes minéraux en présence d'acides minéraux dilués à concentrés, à des températures de 50 à 100°C, de manière à obtenir la chloro-6 benzoxazolone, et on hydrolyse celle-ci, sans ou après l'avoir l'isolée intermédiairement, au moyen d'acides minéraux de concentration moyenne à élevée, à des températures de 100 à 170°C.

2. Procédé selon la revendication 1a, caractérisé en ce qu'on fait réagir la benzoxazolone avec une lessive de blanchiment au chlore d'une concentration pondérale de 13 à 13,5% (qui contient, par litre, environ 160 à 165 g de chlore actif).

3. Procédé selon la revendication 1b, caractérisé en ce qu'on effectue la chloration de la benzoxazolone avec du peroxyde d'hydrogène dans un milieu aqueux acidifié par un acide minéral, qui a une teneur en acide chlorhydrique de 20 à 30 %.

4. Procédé selon la revendication 1, caractérisé en ce qu'on effectue la coupure hydrolytique de la chloro-6 benzoxazolone dans un acide chlorhydrique d'une concentration comprise entre 15 et 37 %.